# EUROPÄISCHE PATENTSCHRIFT

(11) **EP 0 826 654 B1**
(45) Veröffentlichungstag und Bekanntmachung des Hinweises auf die Patenterteilung: **13.01.1999**
(21) Anmeldenummer: 97114428.2
(22) Anmeldetag: 21.08.1997
(51) Int. Cl.: C07C 13/547

(54) **Verfahren zur Herstellung von Indenderivaten**
Process for the preparation of derivatives of indene
Procédé pour la préparation de dérivés d'indène

(30) Priorität: 28.08.1996 DE 19634684
(43) Veröffentlichungstag der Anmeldung: 04.03.1998
(73) Patentinhaber: BASF AKTIENGESELLSCHAFT, 67056 Ludwigshafen (DE)
(72) Erfinder: Müller, Hans-Joachim, Dr., 67269 Grünstadt (DE); Trübenbach, Peter, Dr., 67065 Ludwigshafen (DE); Rieger, Bernhard, Prof. Dr., 89075 Ulm (DE); Wagner, Jürgen Matthäus, 89160 Dornstadt (DE); Dietrich, Ulf, 89079 Ulm (DE)

(56) Entgegenhaltungen:
- EP-A- 0 545 304

## Beschreibung

Verfahren zur Herstellung von Indenderivaten der allgemeinen Formel I 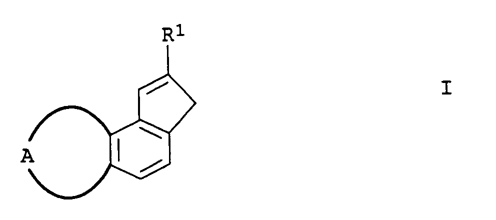 in welcher
- R¹: einen C₁-C₁₀-Alkylrest, einen Phenylrest oder einen C₁-C₄-alkylsubstituierten Phenylrest und
- A: ein Brückenglied, welches zusammen mit den benachbarten Kohlenstoffatomen einen sechsgliedrigen aromatischen oder einen fünf- oder sechsgliedrigen heteroaromatischen oder aliphatischen Ring bildet, an welchen ein weiterer aromatischer Ring anelliert sein kann,
bedeuten.

Indenderivate der allgemeinen Formel I sind wichtige Vorstufen für Metallocenkomplexe, die als Polymerisationskatalysatoren Verwendung finden. Dazu werden die Indenderivate mit Übergangsmetallen, insbesondere mit Zirkoniumsalzen sowie mit metalloceniumionenbildenden Verbindungen zu den entsprechenden Metallocenkomplexen umgesetzt.

Zur Herstellung substituierter Indene sind verschiedene Verfahren bekannt. Üblicherweise wird dabei als Zwischenstufe zunächst ein Indanonderivat hergestellt, welches anschließend durch Reduktion und Dehydratisierung in das entsprechende Indenderivat überführt wird.

In der EP-A1-549 900 wird die Synthese eines substituierten Benzindanons beschrieben. Der Fünfring wird dabei in einer mehrstufigen Synthese durch Umsetzung mit einem Malonester, alkalische Verseifung des Diesters, thermische Decarboxylierung, Chlorierung der verbleibenden Carboxylgruppe und intramolekulare Friedel-Crafts-Acylierung gebildet. Die Synthese ist durch ihre Vielstufigkeit mit großem technischen Aufwand verbunden.

In der EP-A1-567 953 wird eine Synthese von Indanonderivaten beschrieben, wobei der Fünfring durch Umsetzung eines entsprechenden Benzolderivates mit einem substituierten Acrylsäureester in flüssigem Fluorwasserstoff gebildet wird. EP-A1-587 107 beschreibt die Herstellung von 2-Methyl-benzindanon durch Umsetzung von Naphthalin mit Methacrylsäureanhydrid in Gegenwart von BF₃/Fluorwasserstoff. Auch diese Synthesewege sind wegen der schwierigen Handhabung der äußerst toxischen Flußsäure mit erheblichem technischen Aufwand verbunden.

Ein weiterer Syntheseweg für Indanone wird in der EP-A1-545 304 beschrieben. Die Herstellung erfolgt hier durch Umsetzung eines Benzolderivates mit α-Halogenalkylpropionsäurehalogeniden, bevorzugt mit α-Bromisobuttersäurebromid.

Nachteilig an diesen Synthesen ist der komplizierte, vielstufige Herstellprozeß, der große Anfall halogenhaltiger Abfälle sowie teilweise der Einsatz sehr toxischer Einsatzstoffe.

Der vorliegenden Erfindung lag daher die Aufgabe zugrunde, einen einfachen Syntheseweg zur Herstellung von Indenderivaten der allgemeinen Formel I zu finden, der die Nachteile der bekannten Synthesewege überwindet.

Demgemäß wurde ein Verfahren zur Herstellung von Indenderivaten der allgemeinen Formel I 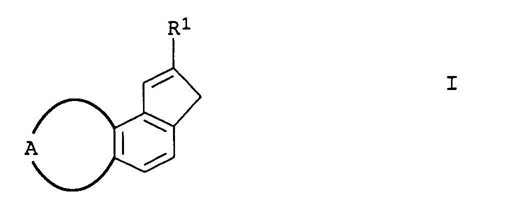 in welcher
- R¹: einen C₁-C₁₀-Alkylrest, einen Phenylrest oder einen C₁-C₄-alkylsubstituierten Phenylrest und
- A: ein Brückenglied, welches zusammen mit den benachbarten Kohlenstoffatomen einen sechsgliedrigen aromatischen oder einen fünf- oder sechsgliedrigen heteroaromatischen oder aliphatischen Ring bildet, an welchen ein weiterer aromatischer Ring anelliert sein kann,
bedeuten, gefunden, welcher dadurch gekennzeichnet ist, daß man eine Verbindung II 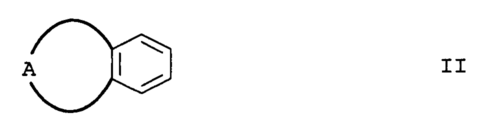 mit einer Verbindung III 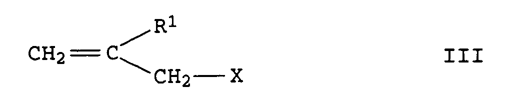 in welcher X Chlor, Brom oder Iod bedeutet, in Gegenwart eines Friedel-Crafts-Katalysators zu einer Verbindung IV 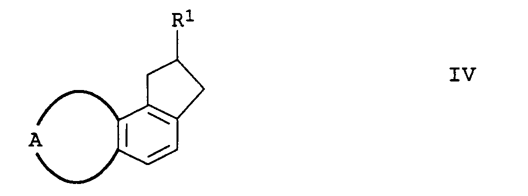 umsetzt und anschließend die Verbindung IV zum Indenderivat I dehydriert.

Der Substituent R¹ wird über die Verbindung III in die Indenderivate eingeführt. R¹ ist vorzugsweise Methyl oder Ethyl, besonders bevorzugt Methyl, jedoch kommen auch andere C₁-C₁₀-Alkylreste, der Phenylrest oder C₁-C₄-alkylsubstituierte Phenylreste in Betracht. Als C₁-C₁₀-Alkylreste kommen neben den obengenannten C₁-C₄-Alkylresten auch die verschiedenen Isomeren Pentyl-, Hexyl-, Heptyl-, Octyl-, Nonyl- und Decylreste in Betracht. Als Phenylreste kommen neben dem bevorzugten unsubstituierten Phenylrest besonders einfach mit Methyl oder Ethyl substituierte Phenylreste in Betracht.

Der Rest X in der Verbindung III kann Chlor, Brom oder Iod bedeuten, bevorzugt ist X Brom.

Das Brückenglied A vervollständigt zusammen mit den zwei benachbarten Kohlenstoffatomen einen sechsgliedrigen aromatischen oder einen fünf- oder sechsgliedrigen heteroaromatischen oder aliphatischen Ring. Die Brückenglieder A können dabei so gewählt werden, daß sowohl substituierte als auch unsubstituierte Ringe entstehen. Als Brückenglieder A, die zu unsubstituierten Ringen führen, kommen beispielsweise die Strukturelemente 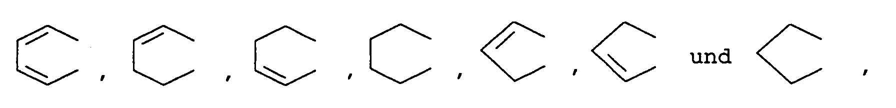 in Betracht. Als Substituenten an diesen Strukturelementen kommen z.B. C₁-C₄-Alkyl, C₁-C₄-Fluoralkyl, Silyl, C₁-C₄-Alkylsilyl, Phenyl und Benzyl in Betracht, wobei als C₁-C₄-Alkylgruppen Methyl, Ethyl, n-Propyl, Isopropyl, n-Butyl, Isobutyl, sec-Butyl und tert-Butyl zu nennen sind.

Bevorzugte Brückenglieder A sind solche, welche die Struktur 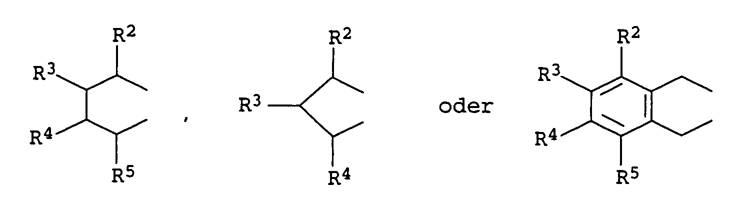 aufweisen, in welcher die Reste R² bis R⁵ Wasserstoff, C₁-C₄-Alkyl, C₁-C₄-Fluoralkyl, Silyl, C₁-C₄-Alkylsilyl, Phenyl oder Benzyl bedeuten, wobei auch hier die oben beschriebenen Alkylreste zu nennen sind.

Bevorzugt ist weiterhin ein Verfahren, welches dadurch gekennzeichnet ist, daß als Verbindung II eine Verbindung IIa 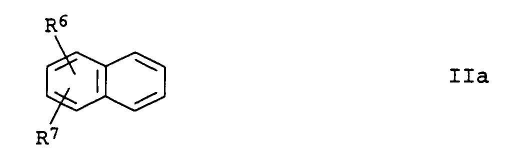 eingesetzt wird, in welcher R⁶ und R⁷ die Bedeutung von R² bis R⁵ haben.

Bevorzugte Reste R⁶ und R⁷ sind Wasserstoff sowie unter den Alkylresten der Isopropylrest. Vorzugsweise ist eine Verbindung IIa jedoch unsubstituiert.

Das erfindungsgemäße Verfahren nutzt als Katalysator im ersten Schritt einen Friedel-Crafts-Katalysator. Als Fiedel-Crafts-Katalysatoren kommen alle üblichen derartigen Katalysatoren in Frage, beispielsweise AlCl₃, AlBr₃, ZnCl₂, FeCl₃, SnCl₄, SbCl₅, TiCl₄, SiCl₄, BF₃ und PCl₅, vorzugsweise wird AlCl₃ verwendet.

Der Katalysator wird vorzugsweise in einer Menge eingesetzt, daß das Molverhältnis von Katalysator zur Verbindung III zwischen 0,1:1 und 1,5:1, vorzugsweise zwischen 0,2:1 und 1,0:1 liegt.

Für das erfindungsgemäße Verfahren sind alle üblichen Lösungsmittel geeignet. In Betracht kommen besonders chlorierte Kohlenwasserstoffe wie Methylenchlorid, 1,2-Dichlorethan, 1,1,2,2-Tetrachlorethan und Chlorbenzol, besonders bevorzugt dient Methylenchlorid als Lösungsmittel.

Nach dem erfindungsgemäßen Verfahren wird die Umsetzung der Verbindungen II und III zu den Verbindungen IV vorzugsweise bei niederen Temperaturen, besonders bevorzugt bei Temperaturen zwischen -80 und 0°C, insbesondere zwischen -40 und -10°C begonnen. Anschließend, d.h. frühestens nach Zugabe aller Reaktanden, kann die Reaktion auch bei etwas höheren Temperaturen, z.B. zwischen 0°C und 50°C, bevorzugt bei 20 - 30°C fortgeführt werden.

Der Druck ist für diesen Verfahrensschritt kein kritischer Parameter, so daß allgemein bei Normaldruck gearbeitet wird.

Für die Dehydrierung des Indanderivates IV zu dem Indenderivat I kommen alle dem Fachmann geläufigen Verfahren für die Dehydrierung von Kohlenwasserstoffketten zu Verbindungen, die in Konjugation zu aromatischen Ringen eine Doppelbindung tragen, in Betracht. Als Katalysatoren eignen sich dafür besonders Platin und Palladiumkatalysatoren, vorzugsweise wird diese Reaktion in Gegenwart von feinverteiltem elementarem Platin, z.B. Platinmohr, durchgeführt.

Bevorzugt wird für die Dehydrierungsreaktion der Verbindung IV zum Indenderivat I als Wasserstoffakzeptor Cyclopenten oder Cyclohexen, besonders bevorzugt Cyclopenten, eingesetzt. Es kommen jedoch für diesen Zweck auch andere cyclische oder offenkettige Olefine in Betracht.

Dieser zweite Reaktionsschritt wird vorzugsweise bei höheren Temperaturen, z.B. bei 50 bis 150°C, besonders bevorzugt bei 80 - 120°C durchgeführt.

Als vorteilhaft hat sich die Durchführung der Reaktion unter Druck, d.h. in einem Autoklaven erwiesen. Dabei wird der Druck im allgemeinen durch die Temperatur und die verwendeten Lösungsmittel vorgegeben. Als Lösungsmittel kommen dabei alle innerten Lösungsmittel, vorzugsweise Hexan, Cyclohexan, Heptan, Oktan, besonders bevorzugt Cyclohexan, in Betracht.

### Beispiele

### Beispiel 1

### Herstellung von 2-Methylbenzindan

Eine Lösung von 20 g (166 mmol) Naphthalin in 50 ml Methylenchlorid wurde zu einer Mischung von 22,13 g AlCl₃ (166 mmol) und 22,41 g 3-Bromisobuten (166 mmol) in 250 ml desselben Lösungsmittels getropft. Die Temperatur wurde dabei auf -10°C gehalten. Nach erfolgter Zugabe wurde die Mischung 12 h bei Raumtemperatur gerührt und anschließend mit 100 ml Wasser hydrolysiert sowie mit 50 ml konz. HCl angesäuert. Nach der Trennung wurde die wäßrige Phase mit Methylenchlorid extrahiert und die vereinigten organischen Phasen mit Wasser ausgeschüttelt. Von der Lösung wurde das Methylenchlorid unter vermindertem Druck abgezogen und der verbleibende Rückstand in Toluol gelöst. Die Feststoffe wurden abgetrennt und die erhaltene Lösung mit K₂CO₃ neutralisiert und mit Na₂SO₄ getrocknet. Das erhaltene Filtrat wurde zur Reinigung von Nebenprodukten an Kieselgel 60 chromatographiert.
Ausbeute: 11 g (36 %)
¹H-NMR-Spektrum (200 MHz, CDCl₃): 1,25 (d, 3H); 1,93 (m, 1H); 2,4 (m, 2H); 2,6 (m, 2H); 6,6 - 7,25 (m, 6H).

### Beispiel 2

### Herstellung von 2-Methylbenzinden

Eine Lösung von 2-Methylbenzindan (19 g, 104 mmol) in 100 ml Cyclohexan sowie 50 ml Cyclopenten wurde im Autoklaven in Gegenwart von frisch erzeugtem Platinmohr 8 Stunden auf 100°C erhitzt. Nach dem Abkühlen und Abziehen des Lösemittels wurde das Rohprodukt an Kieselgel 60 chromatographiert. Nach Abdestillieren des Lösungsmittels wurde das Produkt als Öl erhalten.
Ausbeute: 9,77 g (52 %)

## Patentansprüche

1. Verfahren zur Herstellung von Indenderivaten der allgemeinen Formel I 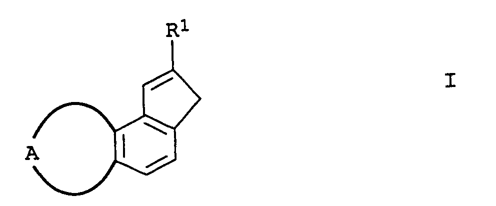 in welcher
R¹ einen C₁-C₁₀-Alkylrest, einen Phenylrest oder einen C₁-C₄-alkylsubstituierten Phenylrest und
A ein Brückenglied, welches zusammen mit den benachbarten Kohlenstoffatomen einen sechsgliedrigen aromatischen oder einen fünf- oder sechsgliedrigen heteroaromatischen oder aliphatischen Ring bildet, an welchen ein weiterer aromatischer Ring anelliert sein kann,
bedeuten, dadurch gekennzeichnet, daß man eine Verbindung II 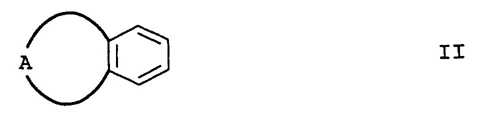 mit einer Verbindung III 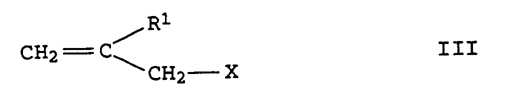 in welcher X Chlor, Brom oder Iod bedeutet, in Gegenwart eines Friedel-Crafts-Katalysators zu einer Verbindung IV 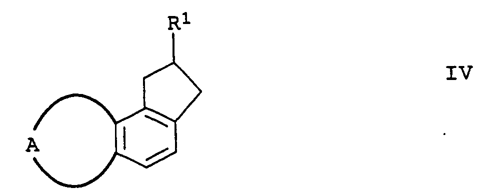 umsetzt und anschließend die Verbindung IV zum Indenderivat I dehydriert.

2. Verfahren nach Anspruch 1, dadurch gekennzeichnet, daß das Brückenglied A die Struktur 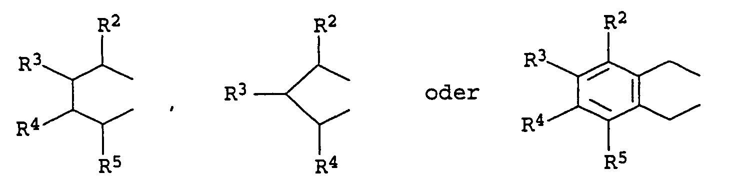 aufweist, in welcher die Reste R² bis R⁵ Wasserstoff, C₁-C₄-Alkyl, C₁-C₄-Fluoralkyl, Silyl, C₁-C₄-Alkylsilyl, Phenyl oder Benzyl bedeuten.

3. Verfahren nach Anspruch 1, dadurch gekennzeichnet, daß als Verbindung II eine Verbindung IIa 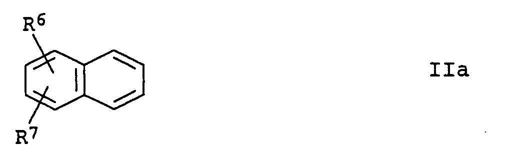 eingesetzt wird, in welcher R⁶ und R⁷ die Bedeutung von R² bis R⁵ haben.

4. Verfahren nach den Ansprüchen 1 bis 3, dadurch gekennzeichnet, daß R¹ Methyl oder Ethyl bedeutet.

5. Verfahren nach den Ansprüchen 1 bis 4, dadurch gekennzeichnet, daß man als Friedel-Crafts-Katalysator AlCl₃ einsetzt.

6. Verfahren nach den Ansprüchen 1 bis 5, dadurch gekennzeichnet, daß man die Umsetzung der Verbindungen II und III zur Verbindung IV bei einer Temperatur von -80 bis 0°C beginnt.

7. Verfahren nach den Ansprüchen 1 bis 6, dadurch gekennzeichnet, daß man bei der Umsetzung der Verbindung IV zum Indenderivat I als Wasserstoffakzeptor Cyclopenten oder Cyclohexen einsetzt.

## Claims

1. A process for preparing indene derivatives of the general formula I 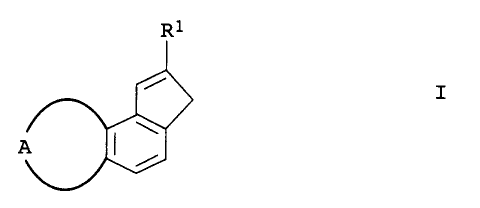 where
R¹ is a C₁-C₁₀-alkyl radical, a phenyl radical or a C₁-C₄-alkyl-substituted phenyl radical and
A is a bridge which, together with the adjacent carbons, forms a six-membered aromatic or a five- or six-membered heteroaromatic or aliphatic ring onto which a further aromatic ring may be fused,
which comprises reacting a compound II 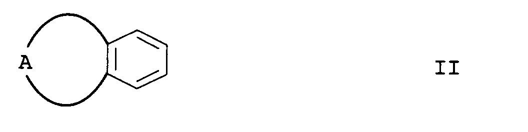 with a compound III 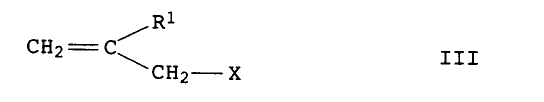 where X is chlorine, bromine or iodine, in the presence of a Friedel-Crafts catalyst to give a compound IV 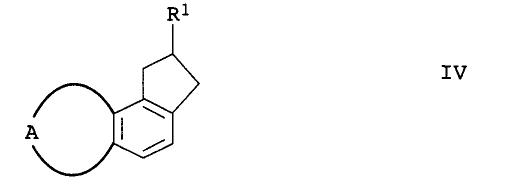 which is then dehydrogenated to the indene derivative I.

2. A process as claimed in claim 1, wherein the bridge A has the structure 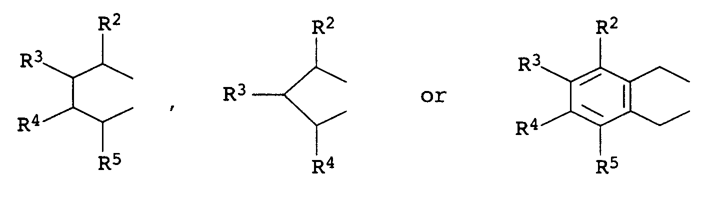 where the radicals R² to R⁵ are each hydrogen, C₁-C₄-alkyl, C₁-C₄-fluoroalkyl, silyl, C₁-C₄-alkylsilyl, phenyl or benzyl.

3. A process as claimed in claim 1, wherein the compound II used is a compound IIa 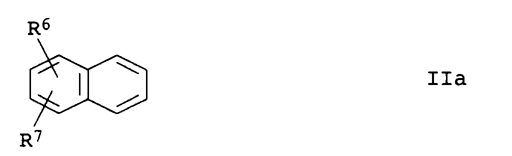 where R⁶ and R⁷ each have the meanings of R² to R⁵.

4. A process as claimed in any of claims 1 to 3, wherein R¹ is methyl or ethyl.

5. The process as claimed in any of claims 1 to 4, wherein the Friedel-Crafts catalyst used is AlCl₃.

6. A process as claimed in any of claims 1 to 5, wherein the reaction of the compounds II and III to give the compound IV is commenced at from -80 to 0°C.

7. A process as claimed in any of claims 1 to 6, wherein the hydrogen acceptor used in the reaction of the compound IV to give the indene derivative I is cyclopentene or cyclohexene.

## Revendications

1. Procédé pour la préparation de dérivés de l'indène de formule générale I 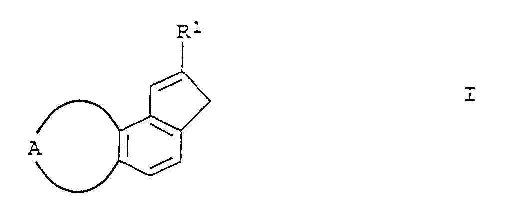 dans laquelle
R¹ représente un groupe alkyle en C1-C10, un groupe phényle ou un groupe phényle à substituant alkyle en C1-C4 et
A représente un pont qui forme avec les atomes de carbone voisins un noyau aromatique à six chaînons ou un noyau hétéroaromatique ou aliphatique à cinq ou six chaînons, qui peut être condensé avec un autre noyau aromatique,
caractérisé par le fait que l'on fait réagir un composé II 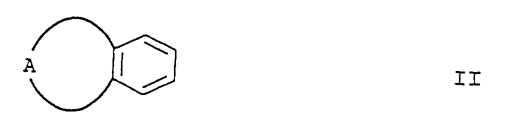 avec un composé III 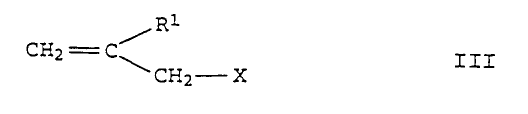 pour lequel X représente le chlore, le brome ou l'iode, en présence d'un catalyseur de Friedel-Crafts, ce qui donne un composé IV 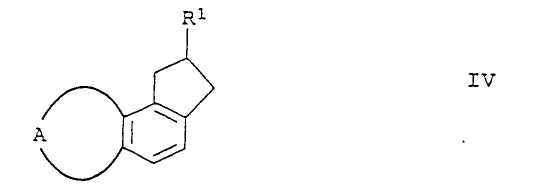 qu'on convertit en le dérivé d'indène I par déshydrogénation.

2. Procédé selon la revendication 1, caractérisé par le fait que le pont A possède la structure 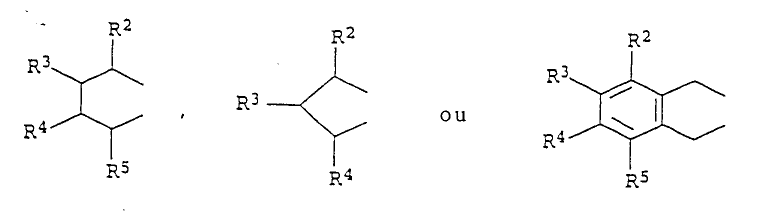 dans laquelle les symboles R² à R⁵ représentent l'hydrogène, des groupes alkyle en C1-C4, fluoralkyle en C1-C4, silyle, (alkyle en C1-C4)silyle, phényle ou benzyle.

3. Procédé selon la revendication 1, caractérisé par le fait que le composé II mis en oeuvre répond à la formule IIa 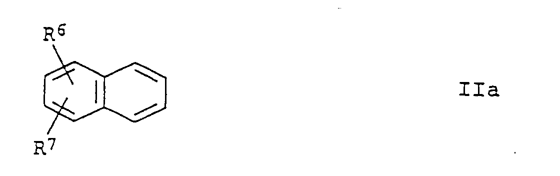 dans laquelle R⁶ et R⁷ ont les significations indiquées pour R² à R⁵.

4. Procédé selon les revendications 1 à 3, caractérisé par le fait que R¹ représente un groupe méthyle ou éthyle.

5. Procédé selon les revendications 1 à 4, caractérisé par le fait que le catalyseur de Friedel-Crafts mis en oeuvre est AlCl₃.

6. Procédé selon les revendications 1 à 5, caractérisé par le fait que l'on fait démarrer à une température de -80 à 0°C la réaction entre les composés II et III donnant le composé IV.

7. Procédé selon les revendications 1 à 6, caractérisé par le fait que, pour la conversion du composé IV en le dérivé de l'indène I, on utilise, en tant qu'accepteur d'hydrogène, le cyclopentène ou le cyclohexène.
